# EUROPEAN PATENT SPECIFICATION

(11) **EP 2 493 490 B1**
(45) Date of publication and mention of the grant of the patent: **19.03.2014**
(21) Application number: 09741264.7
(22) Date of filing: 08.10.2009
(51) Int. Cl.: A61K 36/889, A61K 36/8962, A61P 33/10

(54) **PHARMACEUTICAL OR VETERINARY MEDICAL PREPARATION OBTAINED FROM COCONUT (COCOS NUCIFERA) AND ONION (ALLIUM CEPA) FOR CONTROLLING FLATWORMS (PLATHELMINTHES) AND/OR THREADWORMS (NEMATODES) IN ANIMALS OR HUMANS**
PHARMAZEUTISCHE ODER VETERINÄRMEDIZINISCHE ZUBEREITUNG AUS DER KOKOSNUSS (COCOS NUCIFERA) UND ZWIEBEL (ALLIUM CEPA) ZUR BEKÄMPFUNG VON PLATTWÜRMERN (PLATHELMINTHES) UND/ODER FADENWÜRMERN (NEMATODEN) BEI TIEREN ODER MENSCHEN
PRÉPARATION MÉDICALE PHARMACEUTIQUE OU VÉTÉRINAIRE OBTENUE À PARTIR DE LA NOIX DE COCO (COCOS NUCIFERA) ET DE L'OIGNON (ALLIUM CEPA) POUR LUTTER CONTRE LES VERRES PLATS (PLATHELMINTHES) ET/OU LES VERS RONDS (NÉMATODES) CHEZ LES ANIMAUX OU LES ÊTRES HUMAINS

(43) Date of publication of application: 05.09.2012
(73) Proprietor: Mehlhorn, Heinz, 41468 Neuss (DE); King Saud University, Riyadh 11451 (SA)
(72) Inventor: SCHMIDT, Juergen, 40221 Duesseldorf (DE); ABDEL GHAFFAR, Fathy, Cairo (EG); AL RASHEID, Khaled, Riyadh (SA); QURAISHI, Saleh, Riyadh (SA); AL-FARHAN, Ahmad, Riyadh (SA); SCHMAHL, Guenther, 50735 Koeln (DE)
(74) Representative: Gille Hrabal
(86) International application number: PCT/EP2009/063072
(87) International publication number: WO 2011/042054

(56) References cited:
- DATABASE WPI Week 201003 Thomson Scientific, London, GB; AN 2010-A08585 XP002585050 & IN 188 248 A1 (MARKOSE K N) 31 August 2002 (2002-08-31)
- HUSSAIN A ET AL: "An account of the botanical anthelmintics used in traditional veterinary practices in Sahiwal district of Punjab, Pakistan" JOURNAL OF ETHNOPHARMACOLOGY, ELSEVIER SCIENTIFIC PUBLISHERS LTD, vol. 119, no. 1, 2 September 2008 (2008-09-02), pages 185-190, XP023904703 ISSN: 0378-8741 DOI: 10.1016/J.JEP.2008.06.034 [retrieved on 2008-07-09]
- OLIVEIRA L M B ET AL: "Anthelmintic activity of Cocos nucifera L. against sheep gastrointestinal nematodes" VETERINARY PARASITOLOGY, ELSEVIER SCIENCE, vol. 159, no. 1, 22 January 2009 (2009-01-22), pages 55-59, XP025805188 AMSTERDAM, NL ISSN: 0304-4017 DOI: 10.1016/J.VETPAR.2008.10.018 [retrieved on 2008-10-15] cited in the application
- ABU EL EZZ NADIA M T: "Effect of Nigella sativa and Allium cepa oils on Trichinella spiralis in experimentally infected rats." August 2005 (2005-08), JOURNAL OF THE EGYPTIAN SOCIETY OF PARASITOLOGY , XP009134260 ISSN: 0253-5890 vol. 35, pages 511-523, cited in the application abstract

## Description

The invention relates to a pharmaceutical or veterinary medical preparation for use in controlling flatworms, threadworms and/or intestinal flukes that parasitise the intestine in humans or animals, characterized in that the sole active ingredients against said worms are obtained from coconut (*Cocos nucifera)* and onion *(Allium cepa*).

### Prior art

Threadworms and flatworms are present in almost all animals and humans and spread considerably in animals that are continuously grazing because, in the case of nematodes (= threadworms), the eggs or larvae excreted in the faeces lead to an immediate subsequent infection when they are ingested orally with the feed. Infections also occur very simply in the case of flatworms, even though intermediate hosts are in most cases involved, which are likewise Infected, by the egg- or larvae-containing piles of faeces of affected grazing animals. The worm population thus increases dramatically during the grazing phase, in particular when the animals are close together. Infestation with flatworms and threadworms is therefore a major problem in the rearing of productive livestock, for example ruminants, in particular, because of the worm-related reduction in weight (Mehihorn 2008; Eckert et al. 2008). However, that problem was already known in the old cultures of the Chinese, Egyptians, etc., because few or no worms were found when healthy animals were slaughtered, but a large number of worms was always found in emaciated animals (Leyung 1985). In earlier times, worms were controlled using specific plants which - more out of belief than knowledge - were credited with having specific actions. Specific plants do actually have an anthelmintic action - as has been shown by today's tests. For example, the consumption of male-fern parts, chilli pods or papaya kernels drives away worms. However, many plant constituents are found to be extremely toxic. In the case of the male-fern (*Dryopteris filix-mas*)*,* for example - In particular in the case of an overdose - there can be poisoning with vomiting, dizziness with visual disturbances to blindness, or even cases of death (Schönfelder and Schönfelder 2001 ; Baumler 2007).

Moreover, many of the plant products used in natural medicine have not been sufficiently tested in terms of either their action or their tolerability in accordance with the safety standards that are necessary today, so that they are nowadays no longer used in practice and have been replaced by chemical products (Eckert et al. 2008; Mehlhorn et al. 1993; Rommel et al. 2003, Mehlhorn 2008).

However, aside from the preference for a clearly demonstrated action, chemical substances have several disadvantages:
1. They are in most cases active against only one worm group - that is to say either only against threadworms or only against flatworms.
2. They lead to tissue depositions, and there are therefore relatively long waiting times before meat, milk or eggs can be released for human consumption following control.
3. They are relatively expensive for the customer because the search for new chemical components is extremely expensive.
4. Most of them become unusable after about 20 years of use because resistance occurs, in particular in the case of mass use. Thus, even today, a plurality of different anthelmintics must be used in succession during the grazing season.
5. Chemical products cannot be used by ecofarmers.

For those reasons it is necessary to seek alternatives from the plant sector, especially since plants have successfully fought worms and insects for millions of years and the consumption of useful plants is daily practice for animals.

There are some reports from natural medicine that parts of the garden onion *(Allium cepa)* and of garlic *(Allium sativum)* have anthelmintic activity (Glove 1996; Faijimi and Taiwo 2005). However, a distinction is not made in the natural medical reports between threadworms and flatworms, which occur together but behave completely differently. For example, feeding green leaves of the garden onion to hens in Nigeria is said to help against "helminthiasis = worm diseases" (Faijimi and Taiwo 2005). Abu EI Ezz (2005) reports more specifically that onion oil helps against trichinia infestation in rats. However, it is not clear whether this action is not due to the expelling action that such oils have because of their considerable fat content. There is therefore no definitive proof that onion extracts act on both worm strains.

Although studies of the action of garlic *(Allium sativum),* which is closely related to the garden onion, have shown that extracts exhibited *in vitro* effects against threadworms, it was often not possible to confirm this in *in vivo* tests with affected animals (Burke et al. 2009; Iqbal et al. 2001; Bastidas 1969), or the degree of action was very low unless very large amounts were used (Ayaz et al. 2008). These differences between *in vltro* and *in vivo* tests suggest that the reported positive *in vitro* results are presumably attributable to possible killing effects of the extracting agents (chloroform, acetonitrile, alcohol, etc.).

There are likewise cursory reports from natural medicine of an "anthelmintic action" for coconut (Cocos *nucifera)* (Duke and Wain 1981; Blini and Lira 2009; Amorim and Borba, 1994, 1995). The action of coconut milk was directed against threadworms (*Syphocia*) and tapeworms (*Vampiroiepis*) of naturally infected rodents. In experimental *in vivo* tests by Oliveira et al. (2009), however, coconut exhibited no effects on gastrointestinal threadworms in sheep. Studies by Costa et al. (2008) had already previously brought a similar result - positive effects *in vitro* but *in vivo:* no effects. On balance, this overview of the literature shows that coconut has certain, not precisely defined effects against flatworms.

K.N. Markose, Database WPI, Week 20103, AN 2010-AO8585 XP002585050 + IN188248 discloses the use of onion and coconut oil, besides other plant ingredients, for preparing an ayurvedic preparation for treating burns.

Traditional knowledge digital library, in particular K, Mudaliar, Aavialikkum Amuthamurai Churukkam, Palani Thandayuthapani Devasthanam pubications, Directorate of Indian Systems of Medicine, Chennai, (1^{st} edition 1975), page 53 discloses a formulation AM05/17 "Karuppa Vayukku Ennai" comprising 20 different plant components, inter alia Cocos *nucifera* and *Allium sepa.* The mixture is described to be an anthelmintic, besides other medical indications.

### Literature

Abu EI Ezz NM (2005) Effects of Nigella sativa and Allium cepa oils on Trichinella spira/is in experimentally infected rats. I Egypt Soc Parasitol 35:511-523
Amorim A., Borba HR (1994) Acav Antihelmintica de plantas X Testes in-vivo com extratos brutos de Cocos nucifera Rev Bras Farm 75: 91-92
Amorim A, Borba HR (1995) Acav Antihelmintica XI. Influcencia de extractos brutos nucifera. Rev Bras Farm 76: 98-99.
Athanasiadou S., Eithiori J, Kyriazakis I (2007) Medicinal plants for helminth parasite control: facts and fictions. Animal 19: 1 392-1 400.
Ayaz E, Türel, Gül A., Silmaz O (2008) Evaluation of the anthelminthic activity of garlic (Allium sativum) in mice naturally infected with Aspiculuris tetraptera. Recent Pat Antiinfect Drug Disc 3: 149-152
Bäumler S (2007) Heilpflanzen, Praxis heute. Urban und Fischer, Munich
Bastidas G (1969) Effects of ingested garlic on Necator americanus and Necator caninum. Am J Trop Met Hyg 18: 920-923
Burke IM, Wells 17, Wsey P, Miller IE (2009) Garlic and papaya lack control over gastrointestinal nematodes in goats and lambs. Vet Parositol.
Cosha CTC, Oliveira LMB, Camurga Vasconalos ALF et al. (2009) Atividade antihelminthica de Cocos nucifera. Vet. Parasitol 157: 211-213.
Eckert I, Friedhoff KT, Jahner H, Deplazes P (2008) Lehrbuch der Tiermedizin, 2nd ed. Enke Stuttgart
Fajimi AK, Taiwo AA (2005) Herbal remedies in animal parasitic diseases in Nigeria: A review. African J. Biotechnol. 4: 303-307.
Giove N (1996) Traditional medicine in the treatment of enteroparositosis. Rev. Gastroenterol. Peru 16: 197-202.
Iqbal Z, Khahid-Nadeem Q, Khan MN, Akthar MSS, Waraich FN (2001)In vitro anthelmintic activity of Allium sativum, Zingiber officinale and Ficus religlos, Inl. I. Agricult. Biol. 3: 454-457
Leung AY (1985) Chinese medical plants, Diederichs Publ., Munich.
Mehlhorn H (2008) Encyclopedia of Parasitology. 3rd ed., Springer, New York.
Mehlhorn et al. (1993) Diagnose and Therapie der Parasitosen Haus-, Heim- und Nutztiere. Fischer, Stuttgart
Oliveira LMB et al. (2009) Anthelmintic activity of Cocos nucifera against sheep gastrointestinal nematodes. Vet. Parasitol. 159: 55-59
Rommel et al. (2001) Veterinärmedizinische Parositologie. Parey, Berlin
Schönfelder Schönfelder P (2001) Heilpflanzenführer. Franckh-Kosmos, Stuttgart

### Disclosure of the Invention

Based on the outlined facts, there was a continued great need for an effective plant preparation against flatworms and (in particular simultaneously) against threadworms.

The object underlying the invention was, therefore, to provide a plant preparation for use in controlling infestation with flatworms, threadworms or intestinal flukes that parasitise the intestine in humans or animals.

The inventors have accordingly carried out studies in order to provide such a preparation and have found that, surprisingly, a preparation obtained solely from coconut and onion as active ingredients exhibits an outstanding action against flatworms and threadworms as well as against intestinal flukes that parasitise the intestine. In particular, it has been found that this preparation is effective simultaneously against flatworms (e.g. tapeworms, intestinal flukes and liver flukes) and threadworms (e.g. intestinal worms, roundworms, trichinia, etc.).

The garden onion (*Allium cepa*) and the coconut (*Cocos nucifera*) have not hitherto been used as sole active ingredients in combination for controlling worm infestation.

Surprisingly, it has been found that preparations obtained from onion *(Allium cepa*) and coconut (*Cocos nucifera)* as sole active Ingredients in combination exhibit an outstanding synergistic action both against flatworm infestation and against threadworm infestation in various animals.

The object is therefore achieved by a pharmaceutical or veterinary medical preparation for the use defined in claim 1.

Preferred embodiments of the preparation are defined in claims 2 to 9.

The invention also comprises a kit-of-parts, in which onion preparation and coconut preparation are present separately for simultaneous, successive or alternate administration.

According to the invention, the garden onion *(Allium cepa)* can be used in all varieties, for example the common garden onion, the red onion, the white onion, the green onion and the Spanish onion.

Also suitable according to the invention are the pearl onion/leek *(Allium ampeloprosum*), the shallot (*Allium ascalonium*) and the spring onion *(Allium flstulosum),*

However, because these onion species are more expensive, sensitive species, which are used for "haute cuisine" rather than for other purposes, they are not preferred according to the invention, although they are entirely suitable.

Other species of the genus *Allium* (the genus *Allium* includes more than 500-700 species) are not suitable according to the invention, for example wild garlic (*Allium ursinum*) and chives (*Allium schoenoprasum*)*,* garlic *(Allium sativum),* star of Persia (*Allium cristophii*) and further species mentioned, for example, in Wikipedia under the keyword "Allium", because they do not yield preparations that exhibit the action according to the invention against worms. For wild garlic and chives, this has been demonstrated by the inventors in the examples below.

Whole onions and coconut fruit flesh are used. The coconut is freed in the conventional manner of the coconut milk contained therein, and the hairy brown outer shell and the hard shell (exocarp, mesocarp and endocarp) are removed to leave the white fruit flesh (also known as copra), which is used as the starting material. The onion can likewise be peeled so that the thin papery outer skin is removed.

The onion can then preferably be comminuted in the conventional manner, for example chopped or cut. The coconut flesh can also be chopped or grated. Comminution can also be carried out, for example, by pureeing or mashing.

The plant parts (puree is also referred to as a plant part hereinbelow) so obtained can then be administered directly.

Because there is a problem with perishability here and the fresh preparation in each case is expensive, the resulting plant parts are preferably used in the form of dry preparations, which are obtained, for example, by drying to constant weight in a heating oven at about 40°C, for example, or by lyophilisation.

The resulting dried or lyophilised plant parts can then be administered directly or they can be processed to powders, for example by grinding, micronisation or crushing. The dried or lyophilised plant parts, both in powder form and in the form of pieces, can be administered in the dry state or alternatively can be taken up in water or a suitable solvent (e.g. polyethylene glycol (PEG) or polyethylene carbonate (PC)) as explained hereinbelow, processed to a paste or suspension and then administered. Further auxiliary substances, in particular taste improvers, can be added to the resulting plant parts, as described hereinbelow,

The inventors have also used various extracting agents (ethanol, chloroform, acetonitrile, etc.) to extract onion and coconut and then tested the extracts in vitro for their activity against both flatworms and threadworms.

There can be used as extracting agents organic solvents, for example ethers, e.g. diethyl ether, tert-butyl methyl ether, diisopropyl ether, tetrahydrofuran, dioxane; alcohols, such as ethanol, methanol, isopropanol, n-propanol, butanols, such as tert-butanol, isobutanol, sec-butanol, n-butanol, pentanol, hexanol and other alcohols; halogenated hydrocarbons, for example methylene chloride, chloroform, carbon tetrachloride, tetrachloroethane; glycols, such as tetraethylene glycol, ethylene glycol, propylene glycol; hydrocarbons (alicyclic and cyclic as well as aromatic), for example cyclohexane, pentane, hexane, benzine, petroleum, benzene, chlorobenzene and other substituted benzenes; dipolar aprotic solvents, such as acetonitrile, dimethylformamide, dimethyl sulfoxide and other organic solvents. The solvents can also be used in the form of a mixture of one or more. Water can likewise be added to the solvent or solvent mixture or can be used as the only solvent. A mixture of ethanol and water in a ratio by volume of from 1:10 to 1 0:1 , preferably from 1:5 to 5:1, particularly preferably 1:1, is preferably used for the extraction, because those two solvents are well tolerated and are generally permitted in medicaments.

The extraction is carried out in the conventional manner, the plants being comminuted (e.g. chopped, grated, cut, pureed) as described above and extracted in an extractor (e.g. Soxhlet extractor) or being heated in comminuted form with the solvent In a suitable vessel and then filtered off or extracted, for example by vapour extraction.

The resulting extract can be administered as such. In order to achieve an exact reproducible dose, the extract can preferably be concentrated by evaporation, optionally under reduced pressure, so that concentrates and finally dry powders are obtained.

The two plant species can be extracted together, or the extraction takes place separately and the resulting extracts or concentrates are then optionally mixed in the desired ratio.

Further auxiliary substances, in particular taste improvers, can optionally be added to the resulting extracts or concentrates, as described hereinbelow.

There is a risk, however, that, in the case of the extracts so obtained, solvent residues may remain in the extract. This is normally undesirable because the solvents can be unhealthy or even toxic for the treated animals or humans, and residues may under some circumstances remain in the flesh of the treated animals, rendering it unsuitable for consumption.

Materials obtained from the two plants are preferably prepared as described above by comminution and drying, further treatment steps optionally being carried out.

Comminution is first carried out as described hereinbefore. Immediately thereafter or after drying, further fine comminution can be carried out, if required, for example by grinding, crushing or micronisation. Grinding and/or micronisation are preferably carried out after previously coarsely comminuted (e.g. cut or chopped) plant parts have been dried.

After comminution, the resulting powder can optionally be moistened with water. The amount of water is preferably from 5 to 15 wt.%, particularly preferably about 1 0 wt.%, so that a paste or, where more water is used, a suspension forms.

The paste or suspension is then preferably subjected to ultrasound treatment, preferably for approximately from 1 to 20 minutes, particularly preferably for from 5 to 10 minutes, in a conventional ultrasound bath or by introducing a transducer into the vessel containing the paste/suspension. Homogenisation thereby takes place. In addition, it has been found that the ultrasound treatment yields particularly effective preparations because the plant parts are broken up by the ultrasound and active ingredients contained therein are released so that they can subsequently better be taken up by the treated animals or humans.

The resulting material can then be used directly. However, it can also be dried, for example at 40°C for several hours or days. It can also be lyophilised. Spray-drying is also possible according to the invention. The dried material can be stored in the heating oven until used or alternatively, after reaching the desired degree of drying, it can be cooled and packaged, for example in cans or plastic bags.

The plant parts (fresh, dried, lyophilised, in pieces or in powder form or in the form of a paste or suspension or extract) can be administered individually, i.e. onion and coconut separately, administration taking place either in succession, e.g. at an interval of a few (e.g. from 3 to 10, preferably about 5) minutes, the order being unimportant, or they can be administered together, a mixture in the desired mixing ratio, which is, for example, from 1:10 to 1 0:1 , preferably from about 3:1 to about 1 :3, particularly preferably about 1:1 (by weight), being prepared prior to administration. They can also be administered alternately, for example onion in the mornings and coconut in the evenings, or vice versa.

The preparations for the use according to the invention can contain only the onion and coconut materials obtained as described hereinbefore as such, but they can also contain further conventional constituents, for example pharmaceutically acceptable carriers, flow improvers, binders, etc.

The preparations for the use according to the invention can be used in the form of a powder mixture or mixture of suspension. However, they can also be processed to pellets, dragées, tablets or other forms of administration by conventional techniques with the addition of conventional auxiliary substances. Where they are present in the form of dry powders, they are preferably taken up in solvents such as water or PEG/PC and given to the animals or added to the feed.

In order to improve acceptance, one or more taste improvers, for example milk powder or sugared cocoa, can be added to the mixture or to the individual plant preparations (i.e. coconut and onion separately). According to the invention, the amounts of milk, powder or cocoa are, for example, from 1 to 10 wt.%, preferably from 2 to 3 wt.%, of the total preparation. Other taste improvers can also be added, for example odorous substances from the usual fodder plants.

The dose of the preparations for the use according to the invention is calculated according to the weight and condition of the treated individual and is, for example in the case of powdered plant parts, approximately from 100 to 2000 mg, preferably from 400 to 1200 mg, per kg of body weight.

Depending on the severity of the worm infestation, treatment is usually carried out for from 6 to 10, preferably for about 8, consecutive days, usually once daily.

Administration is preferably oral.

The preparation for the use according to the invention can be used to treat humans and animals, preferably mammals, for example productive livestock, domestic animals and pets, such as sheep, cattle, horses, goats, pigs, mice, rats, dogs, cats, hamsters, guinea pigs, etc., zoo animals, such as elephants, big cats, giraffes, antelopes, zebras, lamas, etc., as well as fish, such as ornamental fish or farmed fish, such as trout, salmon or the like in fish rearing ponds or fish farms, birds or reptiles.

The invention comprises also a pharmaceutical or veterinary medical preparation as described before for use in admixture with animal fodder, in particular concentrate fodder or add-on fodder. The preparation is, in this case, preferably presented in form of pellets comprising the preparation for the use according to the invention and the fodder.

Further, the invention comprises a pharmaceutical or veterinary medical preparation for the use as defined in claim 1 which may be administered in form of dainties to dogs, cats and other animals.

The invention is explained further hereinbelow by means of examples.

### Example 1

Onion and coconut preparations were prepared by the following process.
a) Grinding and optional micronisation of dried onions (without skin) or of coconut fruit flesh.
b) Moistening of the powdered material with water.
c) Ultrasound treatment for 5 to 10 minutes.
d) Drying of the material in a heating oven at 40°C for storage until use.
e) For administration, the dried mass was taken up in water or in a 1:1 mixture of PEG (polyethylene glycol) and PC (polyethylene carbonate), in a ratio of 1:10 (m/m) plant preparation/water.

The suspensions were then administered daily for in each case eight days to experimentally infected mice and naturally infected sheep. Surprisingly, that mixture of coconut and onion was found to kill 100% of both flatworms and threadworms.

The recovery of adult *T. muris* was studied in experimentally infected female laboratory mice (C 57/BL 10 or NMRI) after eight days' oral administration of onion preparation or coconut preparation on its own or in combination, the mice being dissected on day 9 after the start of the test and the worm infestation being studied and compared with untreated controls.

**Onlon preparation:** 2 g of onion powder made up to a total volume of 20 ml with polyethylene glycol (= PEG)/propylene carbonate (= PC) (1:1, v/v).

**Coconut preparation:** 2 g of dried coconut flesh made up to a total volume of 20 ml with PEG/propylene carbonate (1:1, v/v).

This gives:
1 ml: 1 00 mg of onion/or 1 00 mg of coconut
i.e. 0.2 ml/20 g mouse, corresponds to 1000 mg/onion or coconut per 1 kg mouse BW (body weight)
chosen dose: 500 mg/kg mouse BW
i.e. 0.1 ml/20 g mouse.

Treatment was carried out 1 x daily, orally, for 8 days. The results are shown in Table 1.

**Table 1:**

| Mouse No. | Infected with | Administration of/ Target dose | Weight of mouse (g)/ Volume to be administered | Findings |
|---|---|---|---|---|
| 1 | *T. muris* | Infection control | - | 74 worms, normal |
| 2 | *T. muris* | Infection control | - | 77 worms, normal |
| 3 | *T. muris* | Onion/500 mg per kg BW | 19.3 g/ 0.096 ml | 13 worms, no noticeable change under light microscope |
| 4 | *T. muris* | Onion/500 mg per kg BW | 20.5 g/0.103 ml | 18 worms, no noticeable change under light microscope |
| 5 | *T. muris* | Coconut/500 mg per kg BW | 21.0 g/0.105 ml | 7 worms, no noticeable change under light microscope |
| 6 | *T. muris* | Coconut/500 mg per kg BW | 20.2 g/0.101 ml | 11 worms, no noticeable change under light microscope |
| 7 | *T. muris* | Onion and coconut/ each 500 mg per kg BW | 21.8 g/ 0.109 ml + 0.109 ml | 1 worm, male, with disrupted spermiogenesis; damaged |
| 8 | *T. muris* | Onion and coconut/ each 500 mg per kg BW | 20.9 g/ 0.104 ml + 0.104 ml | 0 |

It is clear from the table that, although onion and coconut on their own have a certain action on the threadworms, the combination of onion and coconut exhibits a markedly better, synergistic action.

The recovery of adult *H. microstoma* in experimentally infected female laboratory mice (C 57/BL 10 or NMRI) after eight days' oral administration of onion or coconut preparation on its own or in combination (obtained as described hereinbefore) was studied as described hereinbefore,

### Treatment: 1 x daily, orally, 8 days.

**Table 1 ff:**

| Mouse No. | Infected with | Administration of/ Target dose | Weight of mouse (g)/ Volume to be administered | Findings |
|---|---|---|---|---|
| 9 | *H. microstoma* | Infection control | - | 10 worms, normal |
| 10 | *H. microstoma* | Infection control | - | 9 worms, normal |
| 11 | *H. microstoma* | Onion/500 mg per kg BW | 36.8 g/ 0.184 ml | 3 worms, with immature proglottids |
| 12 | *H*. *microstoma* | Onion/500 mg per kg BW | 40.7 g/ 0.203 ml | 3 worms, with immature proglottids |
| 13 | *H. microstoma* | Coconut/500 mg per kg BW | 37.8 g/0.189 ml | 4 worms, without noticeable change under light microscope |
| 14 | *H. microstoma* | Coconut/500 mg per kg BW | 44.9 g/0.224 ml | 2 worms, without noticeable change under light microscope |
| 15 | *H. microstoma* | Onion and coconut/ each 500 mg per kg BW | 40.3 g/ 0.201 ml + 0.201 ml | 0 |
| 16 | *H. microstoma* | Onion and coconut/ each 500 mg per kg BW | 40.1 g/ 0.201 ml + 0.201 ml | 0 |

It is clear from the table that, although onion and coconut on their own have a certain action on tapeworms, the combination exhibits a markedly increased synergistic effect.

### Example 2

The effectiveness of the combinations coconut preparation with onion preparation and milk powder or coconut preparation with onion preparation, milk powder and cocoa powder against intestinal stages of adult *Trichuris muris* or adult *Hymenolepis microstoma* in the mouse model was studied. The onion and coconut preparations were prepared as described hereinbefore. Administration was effected orally, 1 x daily, for 8 days. On day 9 after the start of the test, the mice were dissected and the worm infestation was studied.

**Table 2:**

| Mouse No. | Parasite | Weight of mouse (g)/ Amount to be admin. (µl) | Treatment | Findings on dissection Number of worms |
|---|---|---|---|---|
| 1 | *T. muris* | 20.2 g/- | Untreated infection control | 64 living worms, normal |
| 2 | *T. muris* | 18.9 g/ 189 µl | 1000 mg coconut + 1000 mg onion + 100 mg milk powder/ kg mouse BW | 0 |
| 3 | *T. muris* | 19.4 g/194 µl | 1000 mg coconut + 1000 mg onion + 100 mg milk powder/ kg mouse BW | 1 female loose in the intestine, moribund |
| 4 | *T. muris* | 19.8 g/198 µl | 1000 mg coconut + 1000 mg onion + 100 mg milk powder/ kg mouse BW | 0 |
| 5 | *T. muris* | 20.4 g/ 204 µl | 1000 mg coconut + 1000 mg onion + 100 mg milk powder/ kg mouse BW | 2 female loose in the intestine, moribund |
| 6 | *T. muris* | 21.5 g/215 µl | 1000 mg coconut + 1000 mg onion + 100 mg milk powder + 100 mg cocoa/ kg mouse BW | 0 |
| 7 | *H. microstoma* | 40.3 g/ - | Untreated infection control | 10 living worms, normal |
| 8 | *H. microstoma* | 41.5 g/ 415 µl | 1000 mg coconut + 1000 mg onion + 100 mg milk powder/ kg mouse BW | 0 |
| 9 | *H. microstoma* | 37.8 g/ 378 µl | 1000 mg coconut + 1000 mg onion + 100 mg milk powder/ kg mouse BW | 0 |
| 10 | *H. microstoma* | 42.8 g/ 428 µl | 1000 mg coconut + 1000 mg onion + 100 mg cocoa/ kg mouse BW | 0 |

It is clear from the table that the combination of onion and coconut preparation has an outstanding action on tapeworms and threadworms. Milk powder and cocoa were added in order to improve the taste and do not impair the anthelmintic action of the preparation according to the invention.

### Example 3

The recovery of adult *T. muris* from experimentally infected C57BL10 mice after seven days' administration of wild garlic powder, onion powder or chive powder was studied. Wild garlic and chive powder were obtained analogously to the procedure described hereinbefore for the preparation of onion powder. Administration was carried out 1 x daily, orally. The dose for each powder was: 2 g + 8 ml PEG/PC (1:1). The doses are based on the powder content (content of dry matter) of the plant in question.

**Table 3:**

| Mouse No. | Dose: mg/kg BW | Findings on dissection day 8 |
|---|---|---|
| 1 | Untreated infection control | 89 worms, vital |
| 2 | Untreated infection control | 83 worms, vital |
| 3 | 500 mg wild garlic powder/kg BW | 81 worms, without noticeable damage |
| 4 | 500 mg wild garlic powder/kg BW | 72 worms, without noticeable damage |
| 5 | 1000 mg wild garlic powder/kg BW | 79 worms, without noticeable damage |
| 6 | 1000 mg wild garlic powder/kg BW | 84 worms, without noticeable damage |
| 7 | 500 mg onion powder/kg BW | 48 worms, sit loosely in the mucosa |
| 8 | 500 mg onion powder/kg BW | 12 worms, sit loosely in the mucosa |
| 9 | 1000 mg onion powder/kg BW | 0 |
| 10 | 1000 mg onion powder/kg BW | 0 |
| 11 | 500 mg chive powder | 68 worms, without noticeable damage |
| 12 | 500 mg chive powder | 77 worms, without noticeable damage |
| 13 | 1000 mg chive powder | 58 worms, without noticeable damage |
| 14 | 1000 mg chive powder | 66 worms, without noticeable damage |

This test shows, in comparison with Example 1, that onion preparation on its own requires twice the dose in order to kill the worms. In addition, the test shows that closely related plant species such as wild garlic and chive do not have an anthelmintic effect.

### Example 4

The in vivo treatment of 6 sheep (about 40 kg) In Schandala (Nile delta, Egypt) was studied. On the day of the start of treatment, the sheep had the following worm eggs in their faeces: *Toxocara, Nematodirus, Trichostrongylus, Haemonchus, Trichuris* and *Monezia.*

### Treatment:

4 animals were treated daily for 8 days, orally, with in each case 60 g of dried onion powder and 60 g of coconut powder (obtained as described hereinbefore) (+ a small amount of milk powder to mask the taste),

2 animals remained untreated as control.

### Results:

On the 9th day after treatment, the faeces of the treated animals was free of worm eggs, while the control animals continued to excrete eggs. They had received dry feed doped with PEG (in order to see whether PEG has an influence). Accordingly, it is demonstrated that:
a) the treatment was successful,
b) PEG itself does not have an effect.

### Example 5

The in vivo treatment of 12 sheep was carried out in Ratingen (Germany). When the faeces were checked beforehand, the following worm eggs were present in the faeces: *Toxocara, Nematodirus, Trichostrongylus, Haemonchus, Trichuris* and *Monezia.*

Treatment as Example 4, checks: day 9 and 20 after the start of treatment.

### Result:

None of the treated animals exhibited further worm eggs in the faeces. The untreated animals, on the other hand, exhibited the previous infestation.

### Example 6

8 sheep were treated in Riyadh. Before the treatment (6 animals, 2 untreated as control), all the animals exhibited the following worms: *Trichuris, Nematodirus, Trichostrongylus, Oesophogstomum* and *Monezia.*

### Results:

The treated animals (8 x 60 g of coconut + 8 x 60 g of onion) no longer exhibited worm eggs in the faeces on the 9th day and on the 20th day after treatment. The untreated animals, on the other hand, exhibited the same amounts of worm eggs as previously. This also shows that excretion of eggs still did not occur a relatively long time after the end of treatment. This means that the treatment had also included preadult stages of the worms.

### Example 7

Mice infected with adult *Echinostoma* intestinal flukes (*Plathelminthes*) were treated as follows: in each case daily with

| Mouse No. | Infected with | Administration | Number of worms on day (dissection) |
|---|---|---|---|
| 1 | *Echinostoma* flukes in the intestine | - (control) | 22/ normal |
| 2 | " | - (control) | 24/ normal |
| 3 | " | 1000 mg onion/kg BW | 12 |
| 4 | " | 1000 mg onion/kg BW | 16 |
| 5 | " | 1000 mg coconut/kg BW | 8 |
| 6 | " | 1000 mg coconut/kg BW | 9 |
| 7 | " | mg onion/ 500 mg coconut/kg BW | 0 |
| 8 | " | " | 0 |

The synergism in the action is marked, as is clearly shwon by the table.

## Claims

1. Pharmaceutical or veterinary medical preparation for use in controlling flatworms, threadworms and/or intestinal flukes that parasitise the intestine in humans or animals, **characterised in that** the sole active ingredients against flatworms, threadworms and/or intestinal flukes that parasitise the intestine are obtained from onions *(Allium cepa)* and coconut (*Cocos nucifera).*

2. Pharmaceutical or veterinary medical preparation for the use according to claim 1, **characterised in that** preparations obtained from onions and coconut are present in admixture.

3. Pharmaceutical or veterinary medical preparation for the use according to claim 1, **characterised in that** preparations obtained from onion and coconut are present in separate units (kit-of -parts) for administration simultaneously or In succession.

4. Pharmaceutical or veterinary preparation for the use according to one or more of claims 1 to 3, additionally comprising one or more further constituents selected from taste improvers and conventional pharmaceutically acceptable auxiliary substances.

5. Combined pharmaceutical or veterinary medical preparation according to claim 1 , 2, 3 and/or 4 for use in a method for simultaneously or individually controlling development stages of flatworms (*plathelminthes*) and/or threadworms (nematodes) in humans or animals, in particular in mammals such as productive livestock and/or pet.

6. Pharmaceutical or veterinary medical preparation for the use according to one or more of the preceding claims for oral administration.

7. Pharmaceutical or veterinary medical preparation for the use according to one or more of the preceding claims which may be used in admixture with animal fodder, in particular concentrate fodder or add-on fodder.

8. Pharmaceutical or veterinary medical preparation for the use according to one or more of the preceding- claims which may be administered in form of dainties to dogs, cats and other animals.

9. Pharmaceutical or veterinary medical preparation according to one or more of claims 1 to 8 for use in a method for the simultaneous or individual control of development stages of flatworms (*plathelminthes*) and/or threadworms (*nematodes*) in humans or animals , in particular in mammals such as productive livestock and/or pets.

## Patentansprüche

1. Pharmazeutische oder veterinärmedizinische Zubereitung zur Verwendung zur Kontrolle von Plattwürmern, Fadenwürmern und/oder Darmsaugwürmern, die im menschlichen oder tierischen Dünndarm parasitieren, **dadurch gekennzeichnet dass** die einzigen Wirkstoffe gegen Plattwürmer, Fadenwürmer und/oder Darmsaugwürmer, die im Dünndarm parasitieren, aus Zwiebeln *(Allium cepa)* und Kokosnuss (*Cocos nuoifera*) erhalten werden.

2. Pharmazeutische oder veterinärmedizinische Zubereitung zur Verwendung nach Anspruch 1, **dadurch gekennzeichnet, dass** aus Zwiebeln und Kokosnuss (*Cocos nuoifera*) erhaltene Zubereitungen im Gemisch vorliegen.

3. Pharmazeutische oder veterinärmedizinische Zubereitung zur Verwendung nach Anspruch 1, **dadurch gekennzeichnet, dass** aus Zwiebeln und Kokosnuss (*Cocos nuoifera*) erhaltene Zubereitungen in getrennten Einheiten (mehrteiliges Kit) zur gleichzeitigen oder aufeinanderfolgenden Verabreichung vorliegen.

4. Pharmazeutische oder veterinärmedizinische Zubereitung zur Verwendung nach einem der Ansprüche 1 bis 3, weiterhin umfassend einen oder mehrere zusätzliche Geschmacksverstärker und herkömmliche, pharmazeutisch verträgliche Hilfsstoffe.

5. Kombinierte pharmazeutische oder veterinärmedizinische Zubereitung nach Anspruch 1, 2, 3 und/oder 4 zur Verwendung bei einem Verfahren zur gleichzeitigen oder aufeinanderfolgenden Kontrolle von Entwicklungsstadien von Plattwürmern (Plathelminthen) und/oder Fadenwürmern (Nematoden) bei Menschen oder Tieren, insbesondere in Säugern, wie Nutzvieh und/oder Haustiere.

6. Pharmazeutische oder veterinärmedizinische Zubereitung zur Verwendung nach einem oder mehreren der vorhergehenden Ansprüche zur oralen Verabreichung.

7. Pharmazeutische oder veterinärmedizinische Zubereitung zur Verwendung nach einem oder mehreren der vorhergehenden Ansprüche, die im Gemisch mit Tierfutter, insbesondere Futterkonzentrat oder Futter-Zusatzstoff verwendet werden können.

8. Pharmazeutische oder veterinärmedizinische Zubereitung zur Verwendung nach einem oder mehreren der vorhergehenden Ansprüche, die in Form von Leckereien für Hunde, Katzen und andere Tiere verwendet werden können.

9. Pharmazeutische oder veterinärmedizinische Zubereitung zur Verwendung nach einem oder mehreren der Ansprüche 1 bis 8 zur Verwendung bei einem Verfahren zur gleichzeitigen oder aufeinanderfolgenden Kontrolle Entwicklungsstadien von Plattwürmern (Plathelminthen) und/oder Fadenwürmern (Nematoden) in Menschen oder Tieren, insbesondere in Säugern, wie Nutzvieh und/oder Haustiere.

## Revendications

1. Préparation médicale pharmaceutique ou vétérinaire pour l'utilisation pour le contrôle des vers plats, des vers de fil et/ou des trématodes intestinaux qui parasitent dans l'intestin des humains ou des animaux, **caractérisé par le fait que** les seule agents actifs contre les plathelminthes, les nématodes et/ou les trématodes intestinaux qui parasitent dans l'intestin sont obtenus à partir des oignions *(Allium cepa)* et de la noix de coco (*Cocos nuoifera*)*.*

2. Préparation médicale pharmaceutique ou vétérinaire pour l'utilisation selon la revendication 1, **caractérisé par le fait que** des préparations obtenues à partir des oignions *(Allium cepa)* et de la noix de coco (*Cocos nuoifera)* sont présentes en mélange.

3. Préparation médicale pharmaceutique ou vétérinaire pour l'utilisation selon la revendication 1, **caractérisée par le fait que** des préparations obtenues à partir des oignions *(Allium cepa)* et de la noix de coco (*Cocos nuoifera*) sont présentes dans des unités séparées (kit à plusieurs parties) pour l'administration simultanément ou successivement.

4. Préparation médicale pharmaceutique ou vétérinaire pour l'utilisation selon l'une des revendications 1 à 3 comprenant en outre un ou plusieurs constituants additionnels sélectionnés parmi les exhausteurs de goût et des substances auxiliaires conventionnels pharmaceutiquement acceptables.

5. Préparation médicale pharmaceutique ou vétérinaire combinée selon la revendication 1, 2, 3 et/ou 4 pour l'utilisation dans un procédé pour le contrôle simultané ou successive des stades de développement des ver plat (des plathelminthes) et/ou des vers de fil (des nématodes) chez les hommes ou les animaux, particulièrement chez les mammifères, tel que le bétail et/ou les animaux domestiques.

6. Préparation médicale pharmaceutique ou vétérinaire pour l'utilisation selon l'une ou plusieurs des revendications précédentes pour l'administration orale.

7. Préparation médicale pharmaceutique ou vétérinaire pour l'utilisation selon l'une ou plusieurs des revendications précédentes qui peut être utilisée en mélange avec le fourrage d'animaux, en particulier le fourrage concentré ou le fourrage supplémentaire.

8. Préparation médicale pharmaceutique ou vétérinaire pour l'utilisation selon l'une ou plusieurs des revendications précédentes qui peut être administrée en forme de friandises pour les chiens, les chats et d'autres animaux.

9. Préparation médicale pharmaceutique ou vétérinaire pour l'utilisation selon l'une ou plusieurs des revendications 1 à 8 pour l'utilisation dans un procédé pour le contrôle simultané ou successive des stades de développement des vers plat (des plathelminthes) et/ou des vers de fil (des nématodes) chez les hommes ou les animaux, particulièrement chez les mammifères, tel que le bétail et/ou les animaux domestiques.
